# EUROPEAN PATENT APPLICATION

(11) **EP 1 347 061 A1**
(43) Date of publication of application: **24.09.2003**
(21) Application number: 01997201.7
(22) Date of filing: 27.11.2001
(51) Int. Cl.: C12Q 1/68, C12N 15/09, G01N 33/50

(54) **METHOD OF QUANTIFYING HIV-1 RNA-DNA HYBRID AND DIAGNOSIS KIT**

(30) Priority: 27.11.2000 JP 2000359886
(71) Applicant: Kato, Shingo, Tokyo 160-8582 (JP)
(72) Inventor: Kato, Shingo, Tokyo 160-8582 (JP)
(74) Representative: Woods, Geoffrey Corlett
(86) International application number: JP0110300
(87) International publication number: WO02042494

(57) **Abstract**

A diagnostic kit for evaluating the progress of an HIV-1-related disease and/or the efficacy of an anti-HIV-1 treatment using the quantity of an HIV-1 RNA-DNA hybrid in a sample as an indicator, comprising: at least one primer pair consisting of a downstream primer having a sequence complementary to a portion of the nucleotide sequence of the constituent RNA of the HIV-1 RNA-DNA hybrid and an upstream primer having a sequence complementary to a portion of the nucleotide sequence of the constituent DNA of the HIV-1 RNA-DNA hybrid; and restriction enzyme by which double-stranded DNA containing the same nucleotide sequence as DNA extended by the primer pair can be cleaved at any specific site in the nucleotide sequence.

SEQUENCE LISTING

<110> KEIO UNIVERSITY

<120> Method for determining amount of HIV-1 RNA-DNA hybrid and diagnosis kits

<130> FP-007PCT

<140>
<141>

<150>JP P2000-359886
<151>2000-11-27

<160> 11

<170> PatentIn Ver. 2.0

<210> 1
<211> 20
<212> DNA
<213> Artificial Sequence

<220>
<223> Description of Artificial Sequence: Primer

<400> 1
cacagtacaa tgtacacatg 20

<210> 2
<211> 20
<212> DNA
<213> Artificial Sequence

<220>
<223> Description of Artificial Sequence: Primer

<400> 2

acagtagaaa aattcccctc 20

<210> 3
<211> 20
<212> DNA
<213> Artificial Sequence

<220>
<223> Description of Artificial Sequence: Primer

<400> 3
ctgttaaatg gcagtctagc 20

<210> 4
<211> 20
<212> DNA
<213> Artificial Sequence

<220>
<223> Description of Artificial Sequence: Primer

<400> 4
aatttctggg tcccctcctg 20

<210> 5
<211> 20
<212> DNA
<213> Artificial Sequence

<220>
<223> Description of Artificial Sequence: Primer

<400> 5
aatttctaga tcccctcctg 20

<210> 6
<211> 24
<212> DNA

<213> Artificial Sequence

<220>
<223> Description of Artificial Sequence: Primer

<400> 6
cacaattaaa actgtgcatt acaa 24

<210> 7
<211> 20
<212> DNA
<213> Artificial Sequence

<220>
<223> Description of Artificial Sequence: Primer

<400> 7
ctgtgcatta caatttctgg 20

<210> 8
<211> 43
<212> DNA
<213> Artificial Sequence

<220>
<223> Description of Artificial Sequence: Primer

<400> 8
taatagvact cactataggg agaaagagca gaagacagtg gca 43

<210> 9
<211> 33
<212> DNA
<213> Artificial Sequence

<220>
<223> Description of Artificial Sequence: Primer

<400> 9
agctatctgt ttgttgttgg gtcttgtaca att 33

<210> 10
<211> 33
<212> DNA
<213> Artificial Sequence

<220>
<223> Description of Artificial Sequence: Primer

<400> 10
cccaacaaca aacagatagc tagcaaatta aga 33

<210> 11
<211> 20
<212> DNA
<213> Artificial Sequence

<220>
<223> Description of Artificial Sequence: Primer

<400> 11
ttgaccactt gccacccatc 20

## Description

### Technical Field

The present invention relates to a method for quantification of an HIV-1 RNA-DNA hybrid and a diagnostic kit.

### Background of the Invention

Human immunodeficiency virus (hereinafter, referred to as "HIV") is the virus which can cause acquired immunodeficiency syndrome (hereinafter, referred to as "AIDS"), and type 1 (HIV-1) and type 2 (HIV-2) are now known. In particular, HIV-1 is the strain which has been epidemic throughout the world and of which various subtypes have been discovered.

The current anti-HIV-1 treatment involves chemotherapy with an anti-viral agent, particularly combination drug therapy in which multiple antiviral agents are administered to a patient (hereinafter, referred to as "combination therapy").

In HIV-1-infected patients, it is generally considered that the plasma HIV-1 RNA level is an indicator of viral activities and the CD4 value (CD4 positive T cell level in the blood) is an indicator of the patient's immunological competence. In the current anti-HIV-1 therapy, the timing of the initiation of the treatment is decided and its efficacy is evaluated based on these two indicators.

However, there have been reported many cases of the anti-HIV-1 combination therapy in which the CD4 value continues to increase while the plasma HIV-1 RNA level remains high. Accordingly, a confusion about the correlation between the anti-retroviral effect and the therapeutic effect has arisen.

Among HIV-1 infected patients who have developed no clinical symptoms for a long period, there are some cases of high RNA level in spite of normal CD4 level. In such cases, a physician cannot often decide whether an anti-HIV-1 treatment should be initiated or not on the patient.

The present inventor focused on the HIV-1 provirus level and improved the method of quantifying HIV-1 provirus level so as to increase the accuracy of its determination. The inventor studied in detail the correlation between the CD4 count and various kinds of HIV-1 level over time on patients under combination therapy. As a result, it was revealed that the provirus level showed a stronger correlation with the rate of change in the CD4 value than did the RNA level (Unexamined Japanese Patent Publication No. 2000-157299). This demonstrated the usefulness of the HIV-1 provirus level as an indicator of the efficacy of an anti-HIV-1 treatment.

However, no indicator has yet been found that is useful in deciding when to initiate or revise an anti-HIV-1 treatment.

Accordingly, an object of the present invention is to provide an indicator useful in deciding when to initiate or revise of an anti-HIV-1 treatment.

Another object of the present invention is to provide a means and methodology useful in measurement of the indicator.

### Disclosure of the Invention

The present inventor focused on the HIV-1 RNA-DNA hybrid level. The inventor quantified the HIV-1 RNA-DNA hybrid levels in peripheral blood mononuclear cells (hereinafter, referred to as "PBMC") from HIV-1-infected patients who have not developed any clinical symptoms for a long period, and studied in detail the correlation between the CD4 value and various kinds of HIV-1 level [RNA level, DNA (provirus) level, and RNA-DNA hybrid level] in the patients over time. As a result, it was found that the RNA-DNA hybrid level showed a stronger correlation with the CD4 value than did the RNA level and the DNA level. The present invention has been accomplished based on this finding. Accordingly, the present invention provides a diagnostic kit for evaluating the progress of an HIV-1-related disease and/or the efficacy of an anti-HIV-1 treatment using the quantity of an HIV-1 RNA-DNA hybrid in a sample as an indicator, comprising: at least one primer pair consisting of a downstream primer having a sequence complementary to a portion of the nucleotide sequence of the constituent RNA of the HIV=1 RNA-DNA hybrid and an upstream primer having a sequence complementary to a portion of the nucleotide sequence of the constituent DNA of the HIV-1 RNA-DNA hybrid; and a restriction enzyme by which double-stranded DNA containing the same nucleotide sequence as DNA extended by the primer pair can be cleaved at any specific site in the nucleotide sequence. The HIV-1-related disease includes, for example, AIDS and AIDS-related syndrome. The diagnostic kit according to the present invention may further comprise a known quantity of DNA capable of competing with the DNA extended by the primer pair.

The present invention also provides a method for DNA amplification by extending DNA using an HIV-1 RNA-DNA hybrid as a template and with at least one primer pair consisting of a downstream primer having a sequence complementary to a portion of the nucleotide sequence of the constituent RNA of the HIV-1 RNA-DNA hybrid and an upstream primer having a sequence complementary to a portion of the nucleotide sequence of the constituent DNA of the HIV-1 RNA-DNA hybrid, comprising the steps of digesting double-stranded DNA in a sample with a restriction enzyme by which double-stranded DNA containing the same nucleotide sequence as DNA extended by the primer pair can be cleaved at a specific site in the nucleotide sequence, and then extending the DNA with the primer pair to achieve DNA amplification.

The present invention additionally provides a method for quantification of an HIV-1 RNA-DNA hybrid in a sample, comprising the steps of amplifying DNA by the method described above and then isolating and detecting the amplified DNA.

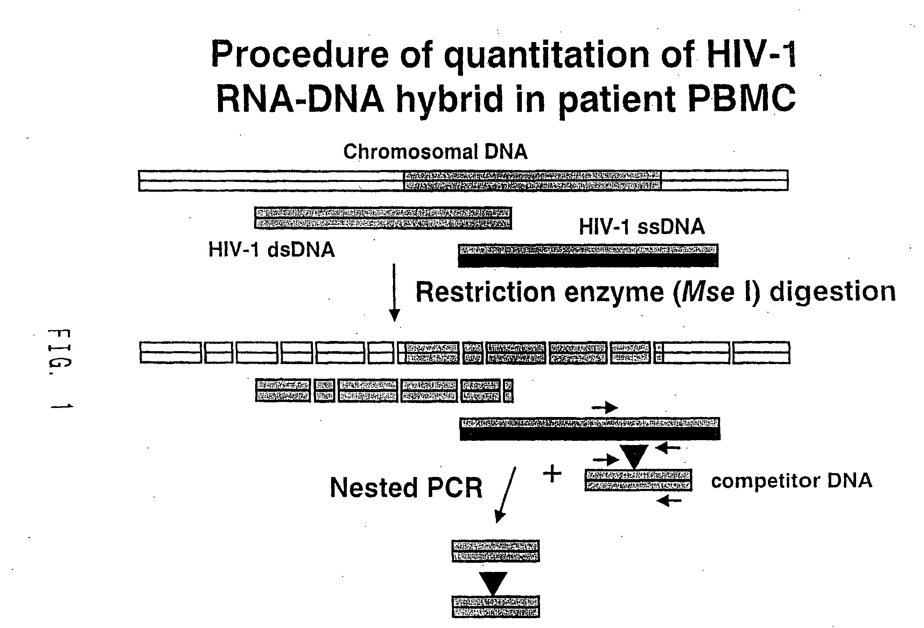

Hereinbelow, the present invention will be described in detail. The procedure of quantification of an HIV-1 RNA-DNA hybrid by the method of the present invention is shown in Fig. 1. In Fig. 1, the solid bars represent RNA chains; the shaded bars represent DNA chains; the solid inverse triangular marks represent deletion sites on a competitor DNA; the arrows represent the directions in which DNA is extended with primers; "dsDNA" represents double-stranded DNA; and "ssDNA" represents a RNA-DNA hybrid.

A sample (e.g., blood, lymph, cerebrospinal fluid, semen, lymph node) is collected from a subject such as a person or patient who has been confirmed to suffer HIV-1 infection or a patient who is under anti-HIV-1 treatment. Cells are isolated from the collected sample by density gradient centrifugation with Ficoll-Paque (Pharmacia), and then DNA is extracted from the cells using QIAamp Blood Kit (QIAGEN). RNA is extracted from the plasma using QIAamp Viral Kit (QIAGEN).

Next, a restriction enzyme is added to cleave the double-stranded DNA. Specifically, 0.5 µg of the DNA is mixed with 5 ng of M13mp18 single-stranded DNA (Takara Shuzo Co., Ltd.), 4 units of restriction enzyme MseI and a MseI buffer, and distilled water is added to make a total volume of 40 µl. The resulting solution is reacted at 37°C for 1 hour. Thereafter, the solution is treated at 65°C for 20 minutes to inactivate the MseI. The restriction enzyme is capable of cleaving the double-stranded DNA having a sequence specifically recognized by the enzyme but is incapable of cleaving a RNA-DNA hybrid having the same sequence. The restriction enzyme used in the present invention should be one having its recognition sites within the target sequence to be amplified in a subsequent PCR. Since HIV-1 virus has a sequence rich in adenine and thymine, the restriction enzyme is preferably one that recognizes a sequence rich in both adenine and thymine and which cleaves double-stranded DNA having the sequence. In the method of the present invention, the DNA to be amplified preferably has a length of several hundred to several thousand of bare pairs (bp). Therefore, a restriction enzyme that recognizes a four-base sequence and which can theoretically cleave double-stranded DNA once every 256 bp is preferably used. Preferred restriction enzyme include MseI, Tsp5091 and RsaI. MseI is most preferred because it exhibits a high enzymatic activity at 37°C.

The nucleic acid sample digested with the restriction enzyme is subjected to PCR, preferably competitive nested PCR (Bruisten et al., AIDS 7 (suppl 2):S15-S20 (1993)) to amplify the HIV-1 RNA-DNA hybrid in the sample.

The method for determination of the HIV-1 RNA-DNA hybrid level by competitive nested PCR will be explained hereinbelow.

Competitive PCR is a method by which two different DNA molecules that can be distinguished by molecular weight, restriction site or the like are simultaneously amplified in a single reaction solution. When DNA to be determined (having an unknown concentration) and a certain amount of a competitor DNA as the internal standard (having a known concentration) are amplified with the same primer pair, the ratio of the amounts of the two reaction products after a plateau phase has been reached reflects the ratio of the initial amounts of the two templates.

Nested PCR is a method in which a target sequence (fragment) to be amplified with the first primer pair is subjected to the first PCR, with the second primer pair being designed as the inner primer set, and then the reaction product of the first PCR is diluted and used as a new template for the second PCR. In the first PCR, an undesired sequence may be amplified in addition to the target sequence. However, the probability that a sequence to which the second primer set can anneal exists in the undesired fragment amplified in the first PCR is extremely low. By performing the two rounds of PCR as stated above, only the target sequence can be amplified selectively.

In the method of the present invention, the competitor DNA which serves as the internal standard for the quantification may be of any kind that is capable of competing with nucleic acids in the target sequence (i.e., a specific region in the HIV-1 RNA-DNA hybrid). It is preferred that the competitor DNA have a length of 2 kb to several kb and most of its internal nucleotide sequence be homologous to the HIV-1 nucleotide sequence. For example, an HIV-1-derived DNA fragment having a deletion, an insertion or a nucleotide substitution introduced therein so as to have a different recognition site of restriction enzyme or a different rate of migration in electrophoresis than the original DNA fragment can be used as the competitor DNA.

The competitor DNA can be produced as follows. Using HIV-1 DNA clone NL4-3 (Adachi et al., JOURNAL OF VIROLOGY, Aug., 1986, pp.284-291) as the starting material, a DNA fragment in which a particular region of the nucleotide sequence corresponding to the target sequence to be amplified with the first primer pair in competitive nested PCR is deleted is prepared by the recombinant PCR method described in Higuchi, PCR Protocols: a guide to methods and application (Academic Press, 1990), pp.177-183.

The competitor DNA can be quantified as follows. The absorbance of a solution of the competitor DNA at 260 nm is measured, and the concentration of the competitor DNA is determined utilizing the conversion relationship stating that the absorption coefficient of DNA is expressed by 1 OD = 50 µg/ml. Two-fold serial dilutions of the solution are prepared, nested PCR is performed using the dilutions, and agarose gel electrophoresis is then performed. The concentration is calculated from the maximum dilution ratio at which the PCR product can be detected, and the concentration determined from the absorbance is corrected.

The HIV-1 RNA-DNA hybrid level is determined by performing competitive nested FCR with the competitor DNA. A known amount (e.g., 10⁴-1 molecule) of the competitor DNA is diluted at an appropriate ratio (2 to 10 fold) and added to a certain amount of a nucleic acid extracted from a subject's sample (e.g., 1-50 µl of a solution prepared by dissolving a nucleic acid extract from 0.1 to 1.0 ml of a blood sample in 20 to 200 µl of sterile water). An appropriate primer pair is used to perform nested PCR. The second primer pair is designed so that the primers are annealed to regions inside of the target sequence to be amplified with the first primer pair. The target sequence to be amplified with the first primer pair may be any region of the HIV-1 virus, as exemplified by a sequence of a specific region of env gene (e.g., a specific region in a highly variable region (V1 to V5 regions, especially V3 region) of gp120 site). The target sequence to be amplified with the first primer pair may have a length of 100 to 4,000 bp, preferably 200 to 400 bp. The target sequence to be ' amplified with the second primer pair may have any length shorter than the target sequence to be amplified with the first primer pair, as exemplified by 50 to 4,000 bp, preferably 200 to 400 bp. The primers may have a length of 18 to 30 bp, preferably 20 to 25 bp. For example, if a sequence of nucleotides 6943-7369 in highly variable region V3 of gp120 site of HIV-1 virus *env* gene is the target sequence, the following two primer pairs can be used.

First primer pair (outer primers):

CACAGTACAATGTACACATG (SEQ ID No. 1)

ACAGTAGAAAAATTCCCCTC (SEQ ID No. 2)

Second primer pair (inner primers):

CTGTTAAATGGCAGTCTAGC (SEQ ID No. 3)

AATTTCTGGGTCCCCTCCTG (SEQ ID No. 4)

When these primer pairs are used, the competitor DNA can be produced as follows. Using HIV-1 DNA clone NL4-3 (Adachi et al., JOURNAL OF VIROLOGY, Aug., 1986, pp.284-291) as the starting material, a DNA fragment of nucleotides 6201-8805 with a deletion of nucleotides 7719-7241 is produced by the recombinant PCR method described in Higuchi, PCR Protocols: a guide to methods and application (Academic Press, 1990) pp. 177-183.

In place of the primer: AATTTCTGGGTCCCCTCCTG (SEQ ID No. 4), either of primers: AATTTCTAGATCCCCTCCTG (SEQ ID No. 5), CACAATTAAAACTGTGCATTACAA (SEQ ID No. 6) and CTGTGCATTACAATTTCTGG (SEQ ID No. 7) may also be used. The procedure and conditions of the PCR may follow the description in Bruisten S. et al., AIDS Res. Hum Retroviruses 1993, 9:259-265. However, since HIV-1 viruses have a high mutation frequency, if PCR is performed under the conditions described in that literature, the rate of amplification of an HIV-1 RNA-DNA hybrid is slower than that of the competitor DNA and the HIV-1 RNA-DNA hybrid level is likely to be underestimated. To determine the HIV-1 RNA-DNA hybrid level precisely without the influence of the mutation of HIV-1 virus, annealing is desirably performed under such conditions that it is first done at a temperature of 48 ± 4°C at least once, preferably three to seven times, and then at a temperature of 64 ± 4°C at least once, preferably 20 to 30 times. It is preferred to employ two different annealing temperatures in each of the first PCR (i.e., PCR with outer primers) and the second PCR (i.e., PCR with inner primers).

The reaction product of the PCR is separated by electrophoresis and detected by ethidium bromide staining. The HIV-1 RNA-DNA hybrid level is calculated from the known competitor DNA level based on the intensity ratio between the band of the amplified competitor DNA and the band intensity of the amplified HIV-1 RNA-DNA hybrid. In practical applications, the determined levels are usually corrected using a known level of wild type HIV-1 DNA as the standard.

In order to compare the correlation between the HIV-1 RNA-DNA hybrid level and the CD4 value with the correlation between the virion RNA level or HIV-1 provirus DNA level and the CD4 value, it is advisable to determine the virion RNA level, the HIV-1 provirus DNA level and the CD4 value on the same blood sample.

The virion RNA level and the HIV-1 provirus DNA level can be determined by the methods described in Unexamined Japanese Patent Publication No. 2000-157299.

The CD4 value can be determined by laser flow cytometry after reacting lymphocytes with the OKT4 antibody.

In addition, the infective HIV-1 level may also be determined. The infective HIV-1 level can be determined by the plaque hybridization method described in Kato et al., J. Virol. Methods 72:1-7 (1998).

As is demonstrated by the examples below, in the blood samples from HIV-1-infected patients who have not developed any clinical symptoms for a long period, the HIV-1 RNA-DNA hybrid level showed a stronger correlation with the CD4 value than did the HIV-1 RNA level and the HIV-1 provirus DNA level. This means that the HIV-1 RNA-DNA hybrid level is a good indicator of the timing on which an anti-HIV-1 treatment should be initiated. The HIV-1 RNA-DNA hybrid level is also useful as a good indicator of when the anti-HIV-1 treatment should be revised.

The present invention also includes a diagnostic kit for evaluating the progress of an HIV-1-related disease and/or the efficacy of an anti-HIV-1 treatment using the quantity of an HIV-1 RNA-DNA hybrid in a sample as an indicator, comprising: at least one primer pair consisting of a downstream primer having a sequence complementary to a portion of the nucleotide sequence of the constituent RNA of the HIV-1 RNA-DNA hybrid and an upstream primer having a sequence complementary to a portion of the nucleotide sequence of the constituent DNA of the HIV-1 RNA-DNA hybrid; and a restriction enzyme by which double-stranded DNA containing the same nucleotide sequence as DNA extended by the primer pair can be cleaved at any specific site in the nucleotide sequence. The primers include, for example, the first primer pair (outer primers) and the second primer pair (inner primers) as described above and any combinations thereof. The restriction enzymes include, for example, those restriction enzymes described above (e.g., MseI, Tsp5091, RsaI). The diagnostic kit according to the present invention may further comprise a known quantity of DNA capable of competing with the HIV-1 RNA-DNA hybrid, as described above. The diagnostic kit may further comprise dNTP mixture, reaction buffer, DNA polymerase and the like. To reduce any effects of base pair mismatch between the primers and the HIV-1 DNA to be tested, the concentration of magnesium ions in the reaction buffer (normally 1.5 mM) is preferably increased to between 3.0 and 6.0 mM, preferably around 4 mM.

In the diagnostic kit, the components of the kit may be accommodated in a container or containers (e.g., vial, tube) separately or in combination or in a group, and the containers may be accommodated in a single carrying means which is partitioned so as to hold the containers in a group.

This specification includes part or all of the contents as disclosed in the specification and/or drawings of Japanese Patent Application No. 2000-359886 which is a priority document of the present application.

### Brief Description of the Drawings

Fig. 1 is a schematic illustration showing the procedure of quantifying HIV-1 RNA-DNA hybrid according to the method of the present invention;
Fig. 2 shows the time courses of changes in CD4 value and HIV-1 RNA level in subject H2;
Fig. 3 shows the time courses of changes in CD4 value and HIV-1 RNA level in subject H31;
Fig. 4 shows the time courses of changes in CD4 value and HIV-1 RNA level in subject C27;
Fig. 5 shows the time courses of changes in CD4 value and HIV-1 RNA level in subject H67;
Fig. 6 shows the time courses of changes in CD4 value and HIV-1 RNA level in subject H310;
Fig. 7 shows the time courses of changes in CD4 value and HIV-1 RNA level in subject H200;
Fig. 8 shows the time courses of changes in CD4 value, HIV-1 DNA level and HIV-1 RNA-DNA hybrid level in subject H2;
Fig. 9 shows the time courses of changes in CD4 value, HIV-1 DNA level and HIV-1 RNA-DNA hybrid level in subject H31;
Fig. 10 shows the time courses of changes in CD4 value, HIV-1 DNA level and HIV-1 RNA-DNA hybrid level in subject C27;
Fig. 11 shows the time courses of changes in CD4 value, HIV-1 DNA level and HIV-1 RNA-DNA hybrid level in subject H67;
Fig. 12 shows the time courses of changes in CD4 value, HIV-1 DNA level and HIV-1 RNA-DNA hybrid level in subject H310;
Fig. 13 shows the time courses of changes in CD4 value, HIV-1 DNA level and HIV-1 RNA-DNA hybrid level in subject H200; and
Fig. 14 shows the results of Pearson's correlation analysis between each of HIV-1 DNA level, HIV-1 RNA-DNA hybrid level and HIV-1 RNA level and CD4 value on fourteen (14) cases.

### Best Mode for Carrying Out the Invention

Hereinbelow, the present invention will be described more specifically by the following examples. However, the invention should not be limited by these examples.

### [Example 1]

Fourteen HIV-1-infected out-patients at a hospital in Tokyo who had not developed any clinical symptoms for a long period and who had not received any anti-HIV-1 combination therapy were employed as test subjects. DNA and RNA were extracted from the peripheral blood. The plasma virion RNA level was determined with AMPLICOR (Roche), and the provirus level and the HIV-1 RNA-DNA hybrid level were determined by competitive nested PCR. The CD4 value was determined by laser flow cytometry after the reaction of lymphocytes with the OKT4 antibody.

### Methods

### 1. Production of competitor HIV-1 DNA fragment

Using HIV-1 DNA clone NL4-3 (Adachi et al., JOURNAL OF VIROLOGY, Aug., 1986, pp.284-291) as the starting material, PCR was performed with a primer pair:
TAATAGVACTCACTATAGGGAGAAAGAGCAGAAGACAGTGGCA (SEQ ID No. 8) and AGCTATCTGTTTGTTGTTGGGTCTTGTACAATT (SEQ ID No. 9) to synthesize a DNA fragment composed of nucleotides 6201-7118 and 7242-7252 of the HIV-1 DNA clone and T7 promoter. PCR was also performed with a primer pair:
CCCAACAACAAACAGATAGCTAGCAAATTAAGA (SEQ ID No. 10) and TTGACCACTTGCCACCCATC (SEQ ID No. 11) to synthesize a DNA fragment composed of nucleotides 7109-7118 and 7242-8805 of the HIV-1 DNA clone. PCR was performed with these two DNA fragments and a primer pair:
TAATAGVACTCACTATAGGGAGAAAGAGCAGAAGACAGTGGCA (SEQ ID No. 8) and TTGACCACTTGCCACCCATC (SEQ ID No. 11) to combine the DNA fragments, thereby synthesizing a DNA fragment composed of nucleotides 6201-8805 of the HIV-1 DNA clone with a deletion of nucleotides 7119-7241 and an addition of T7 promoter upstream of the fragment. Thus, the competitor DNA for use in this experiment was produced. The conditions for PCR were: 20 cycles, each of consisting 15 seconds at 94°C, 30 seconds at 64°C and 60 seconds at 72°C, with 0.5 unit of Taq DNA polymerase (Perkin-Cetus). The concentration of the competitor DNA was determined by measuring the absorbance at 260 nm and performing calculation utilizing the conversion relationship stating that the absorption coefficient of DNA is expressed by 1 OD = 50 µg/ml. Two-fold serial dilutions of the solutions were prepared, used to perform nested PCR, and then subjected to agarose gel electrophoresis. The concentration was calculated at the maximum dilution ratio at which the PCR product can be detected, and the concentration determined from the absorbance was corrected.

### 2. Determination of HIV-1 DNA level by competitive nested PCR

The HIV-1 DNA level was determined as follows. 0.5 µg of DNA derived from PBMC of HIV-1-infected patients was mixed with 50 copies of the competitor DNA, and the resulting mixture was subjected to PCR with a primer pair: CACAGTACAATGTACACATG (SEQ ID No. 1) and ACAGTAGAAAAATTCCCCTC (SEQ ID No. 2). The conditions for the PCR were: a 5-minute incubation at 97°C, followed by 5 cycles, each consisting of 15 seconds at 97°C, 30 seconds at 48°C and 60 seconds at 72°C and then 25 cycles, each consisting of 15 seconds at 94°C, 30 seconds at 60°C and 60 seconds at 72°C. The reaction solution contained 0.5 unit of Taq DNA polymerase (Perkin-Cetus) and an ordinary buffer supplemented with 4 mM magnesium chloride in a total volume of 100 µl. After the reaction was completed, a 2-µl aliquot was sampled from the reaction solution and subjected to the second PCR with a primer pair: CTGTTAAATGGCAGTCTAGC (SEQ ID No. 3) and AATTTCTGGGTCCCCTCCTG (SEQ ID No. 4). The conditions for the PCR were: a 5-minute incubation at 94°C, followed by 5 cycles, each consisting of 15 seconds at 94°C, 30 seconds at 48°C and 60 seconds at 72°C and then 20 cycles, each consisting of 15 seconds at 94°C, 30 seconds at 64°C and 60 seconds at 72°C. The reaction solution contained 0.5 Unit of Taq DNA polymerase (Perkin-Cetus) and an ordinary buffer supplemented with 4 mM magnesium chloride in a total volume of 100 µl. A 20-µl aliquot was sampled from the PCR reaction solution and subjected to electrophoresis on 2% agarose gel at 120 V for 1 hour. The band derived from the competitor DNA and the band derived from the HIV-1 DNA of individual HIV-1-infected patients were quantified for intensity using a CCD camera (Gel Print 2000i/VGA, Genomic Solutions) and an image analysis software package (Intelligent Quantifier, Genomic Solutions). The number of copies of HIV-1 DNA present in the original 0.5 µg DNA from PBMC of the HIV-1-infected patients was calculated by the formula: 50 x (intensity of band from HIV-1 DNA in patient)/(intensity of band from the competitor DNA).

### 3. Determination of HIV-1 RNA-DNA hybrid level with MseI by competitive nested PCR

The HIV-1 RNA-DNA hybrid level was determined as follows. 0.5 µg of DNA derived from PBMC of HIV-1-infected patients was mixed with 5 ng of M13mp18 single-stranded DNA (Takara Shuzo Co., Ltd.), 4 units of MseI (New England BioLab) and MseI buffer. Distilled water was added to the mixture to make a total volume of 40 µl, and the resulting solution was reacted at 37°C for 1 hour. Thereafter, the reaction solution was treated at 65°C for 20 minutes to inactivate MseI. The reaction solution was used to perform the competitive nested PCR described in Section 2.

### 4. Patients

Fourteen HIV-1-infected out-patients at a hospital in Tokyo who had not developed any clinical symptoms for a long period and who had not received treatment with an anti-retroviral agent were used as test subjects. After obtaining informed consent from the patients, 10 ml of peripheral blood was collected from the individual patients. As the anti-coagulant, sodium citrate was used.

### 5. Preparation of nucleic acids

PBMC were isolated by density gradient centrifugation with Ficoll-Paque (Pharmacia), and DNA was prepared from the cells using QIAamp Blood Kit (QIAGEN). The peripheral blood was also centrifuged to separate the plasma, and RNA was prepared from the plasma using QIAamp Viral RNA Kit (QIAGEN). The DNA and RNA were separately dissolved in purified water or an EDTA-containing buffer and stored at -20°C until just before use.

### 6. Determination of CD4 positive T cell counts (CD4 value)

The determination of CD4 positive T cell counts was commissioned to S.R.L., where the peripheral blood collected from individual HIV-1-infected patients was treated with the fluorescent OKT4 antibody and CD4 positive T cell counts was determined by fluorescent laser cytometry.

### Results

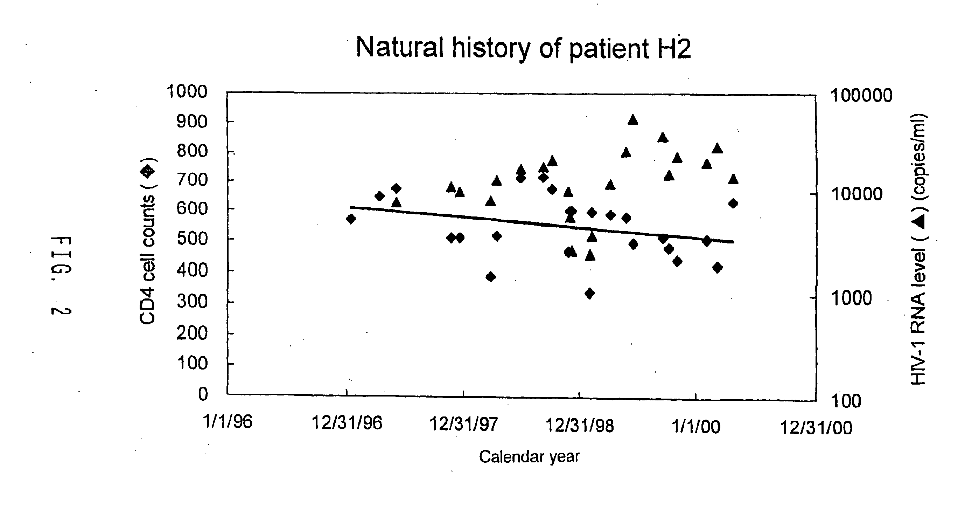

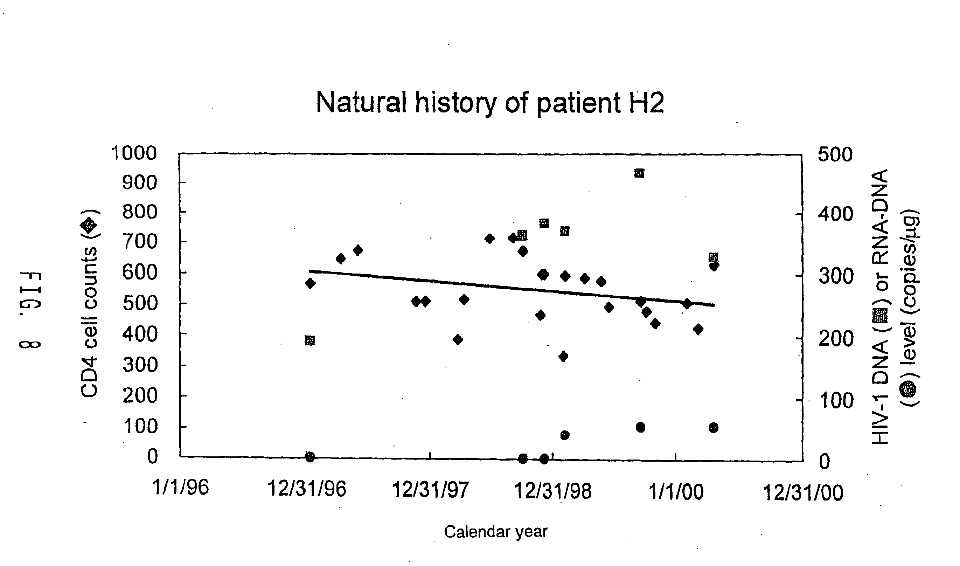

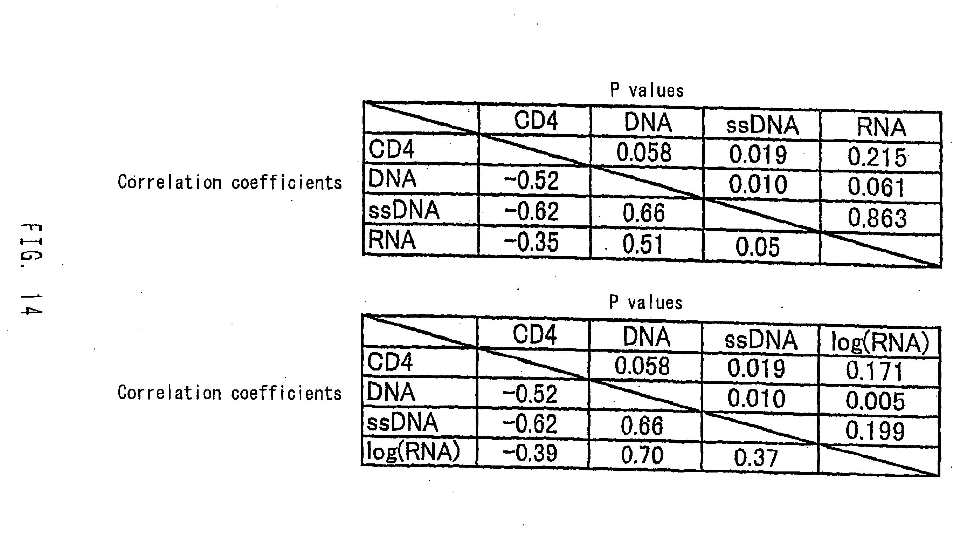

The results of the determination of CD4 value (cells/µl) and HIV-1 RNA level (copies/ml) on six subjects (H2, H31, C27, H67, H310 and H200) are shown in Figs. 2 to 7, respectively. The results of the determination of CD4 value (cells/µl), HIV-1 DNA level (copies/µg) and HIV-1 RNA-DNA hybrid level (copies/µg) on the same subjects are shown in Figs. 8 to 13, respectively. The results of the Pearson's correlation analysis between HIV-1 DNA level, HIV-1 RNA-DNA hybrid level and HIV-1 RNA level and CD4 value on fourteen cases based on the average values for the last two years are shown in Fig. 14.

In Figs. 2 to 7, solid diamond-shaped marks represent CD4 values (cells/µl), and solid triangular marks represent HIV-1 RNA levels (copies/ml).

In Figs. 8 to 13, solid diamond-shaped marks represent CD4 values (cells/µl), solid square marks represent HIV-1 DNA levels (copies/µg), and solid circular marks represent HIV-1 RNA-DNA hybrid levels (copies/µg).

In Fig. 14, "CD4" represents the CD4 value, "DNA" represents the HIV-1 DNA level, "ssDNA" represents the HIV-1 RNA-DNA hybrid level, and "RNA" represents the HIV-1 RNA level.

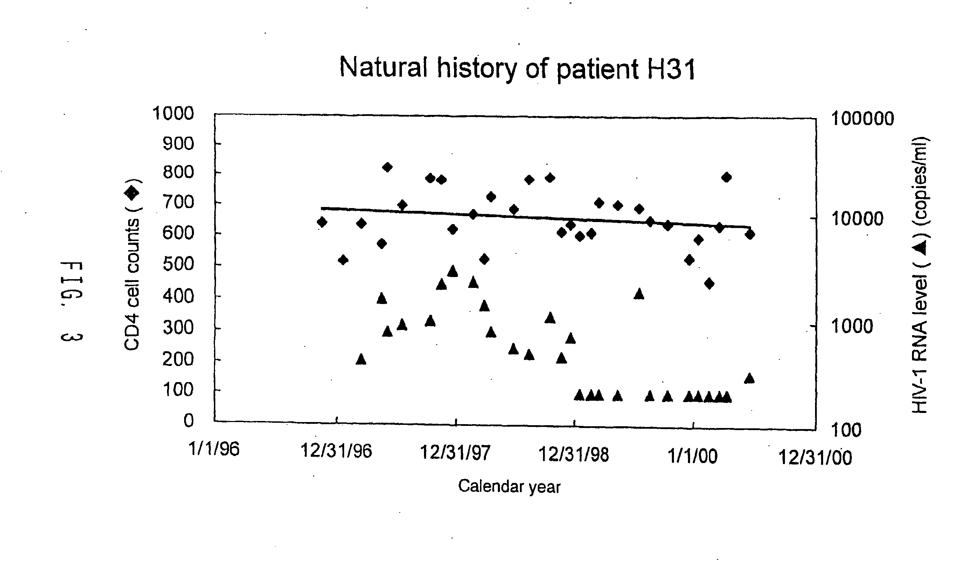

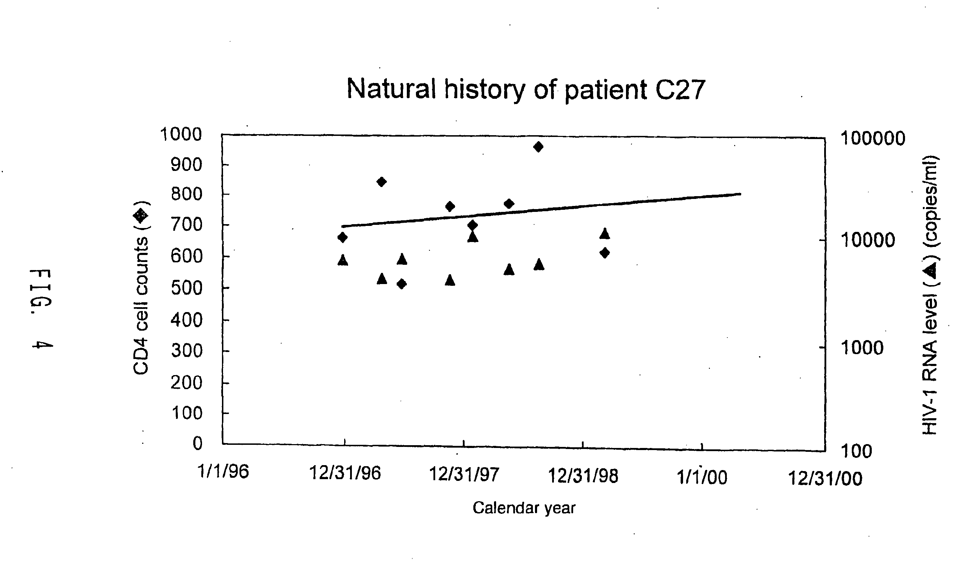

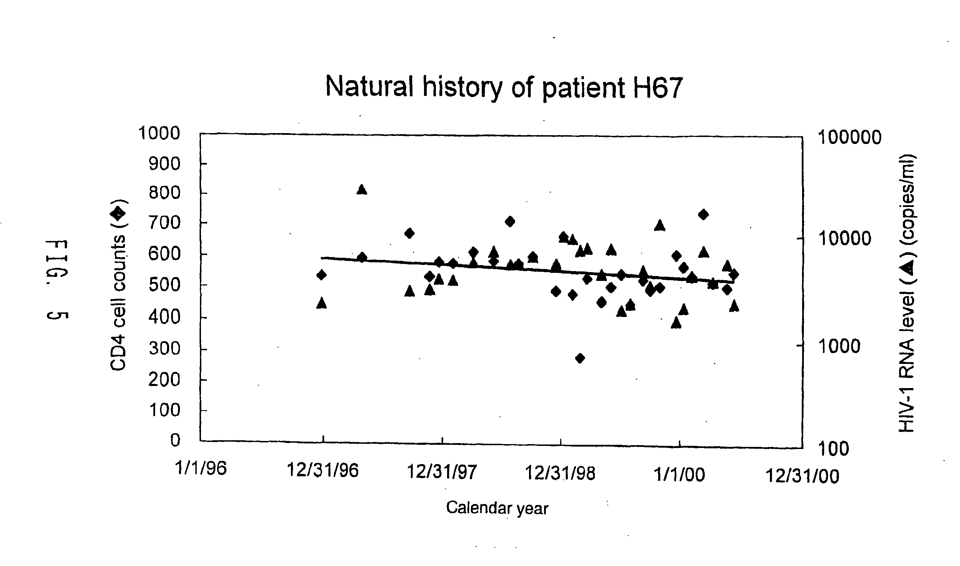

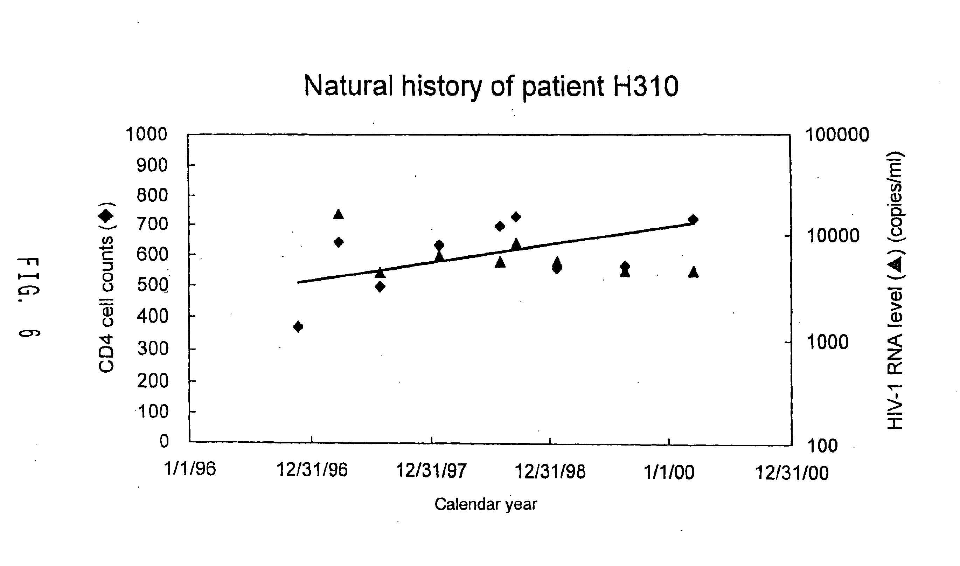

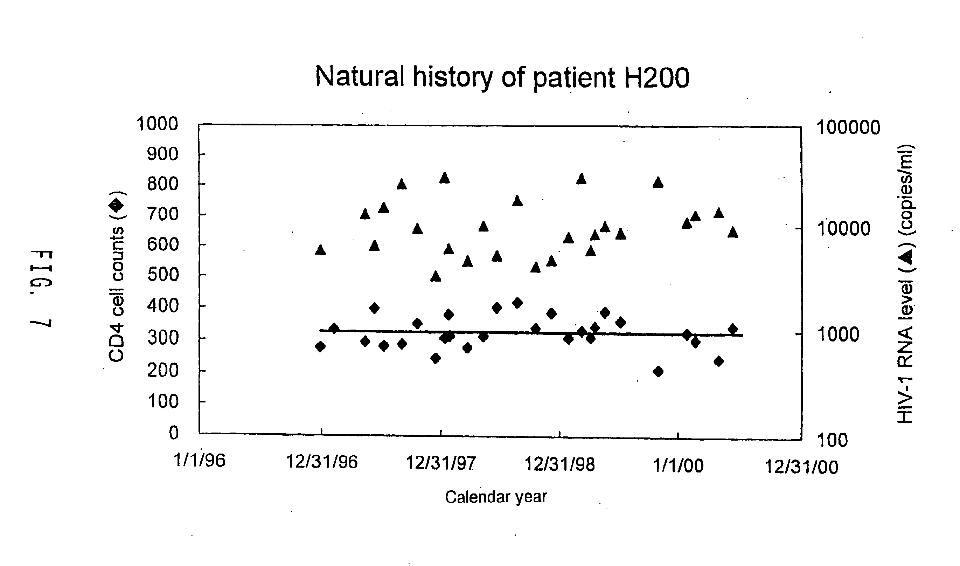

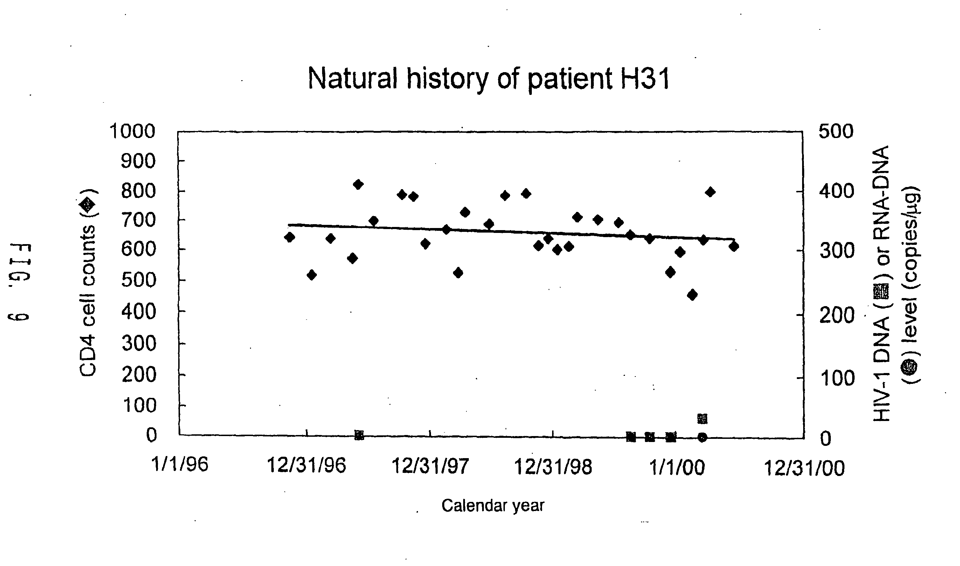

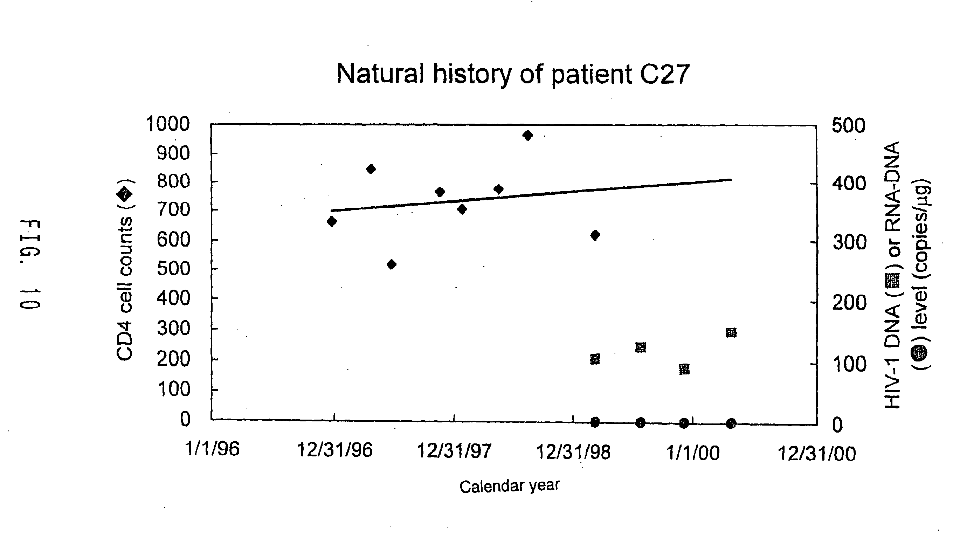

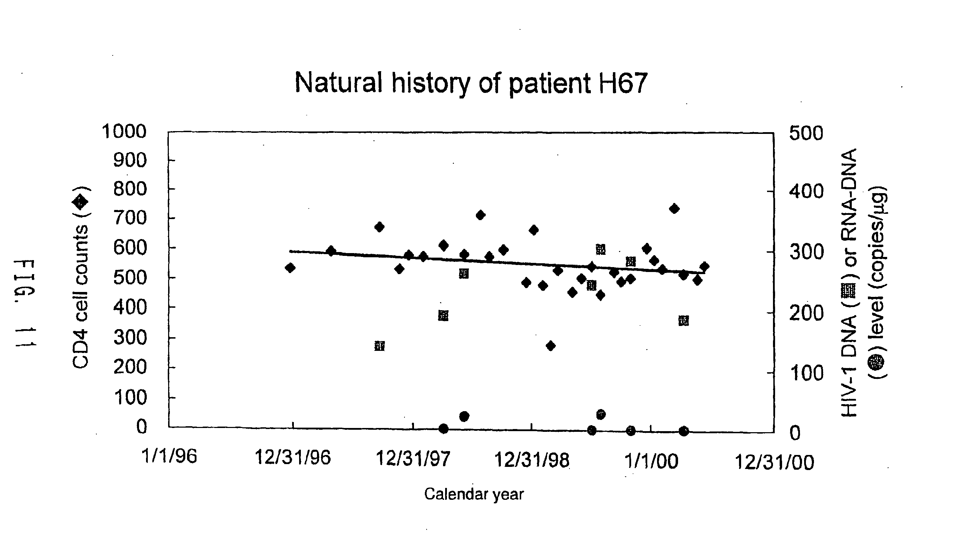

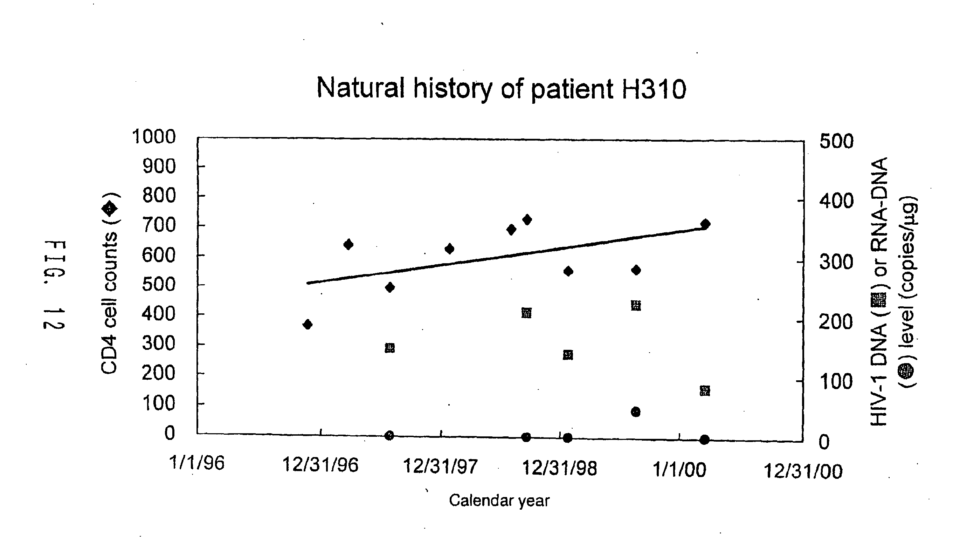

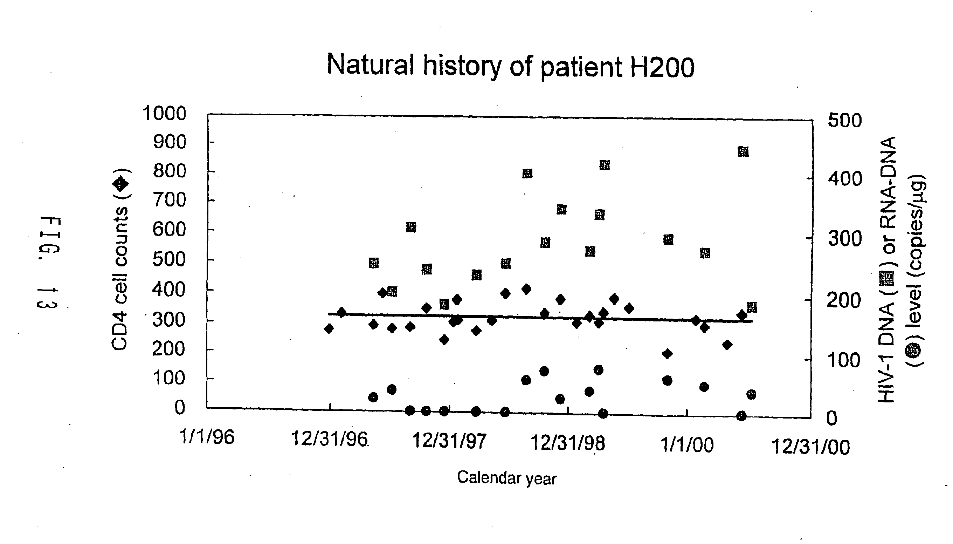

As shown in Figs. 2 to 14, the HIV-1 RNA-DNA hybrid level showed a good correlation with the CD4 value. In the cases where the CD4 value was low and the HIV-1 RNA level was high, the HIV-1 RNA-DNA hybrid level was high [H2 (Figs. 2 and 8) and H200 (Figs. 7 and 13)]. In the cases where the CD4 value was high and the HIV-1 RNA level was low, the HIV-1 RNA-DNA hybrid level was low [H31 (Figs. 3 and 9)]. In the cases where the HIV-1 RNA level was high even though the CD4 value was high, the HIV-1 RNA-DNA hybrid level was low [C27 (Figs. 4 and 10), H67 (Figs. 5 and 11) and H310 (Figs. 6 and 12)]. As a result of Pearson's correlation analysis, the coefficient of correlation between the CD4 value and the HIV-1 RNA level was -0.35 (the coefficient of correlation between the CD4 value and the logarithm of the HIV-1 RNA level was -0.39) and the coefficient of correlation between the CD4 value and the HIV-1 DNA level was -0.52, whereas the coefficient of correlation between the CD4 value and the HIV-1 RNA-DNA hybrid level was -0.62 (Fig. 14). In a significance test of these correlation coefficients, only the coefficient of correlation between the CD4 value and the HIV-1 RNA-DNA hybrid level was found to be statistically significant (p=0.01).

From these results, it is demonstrated that the HIV-1 RNA-DNA hybrid level has a stronger correlation with the CD4 value than the HIV-1 RNA level and the HIV-1 DNA level.

### Discussion

It is concluded that the HIV-1 RNA-DNA hybrid level is a good indicator of the progress of HIV-1 infection. This would be because an HIV-1 RNA-DNA hybrid is a reverse transcription intermediate produced immediately after viral infection of cells and therefore the concentration of the hybrid represents the degree of infection in an infected individual. Probably, if the HIV-1 RNA-DNA hybrid level is lower than the detection limit, there would be no need to initiate an anti-retrovirus treatment or to revise the anti-retrovirus treatment being currently applied.

All publications, patents and patent applications cited herein are incorporated herein by reference in their entirety.

### Industrial Applicability

According to the present invention, an indicator useful in deciding the timing of initiation or revision of an anti-HIV-1 treatment is provided. Means and methodology useful in determining the indicator are also provided.

### Sequence Listing Free Text

SEQ ID No. 1 shows the nucleotide sequence of a primer.
SEQ ID No. 2 shows the nucleotide sequence of another primer.
SEQ ID No. 3 shows the nucleotide sequence of yet another primer.
SEQ ID No. 4 shows the nucleotide sequence of still another primer.
SEQ ID No. 5 shows the nucleotide sequence of another primer.
SEQ ID No. 6 shows the nucleotide sequence of yet another primer.
SEQ ID No. 7 shows the nucleotide sequence of still another primer.
SEQ ID No. 8 shows the nucleotide sequence of yet another primer.
SEQ ID No. 9 shows the nucleotide sequence of another primer.
SEQ ID No. 10 shows the nucleotide sequence of still another primer.
SEQ ID No. 11 shows the nucleotide sequence of yet another primer.

## Claims

1. A diagnostic kit for evaluating the progress of an HIV-1-related disease and/or the efficacy of an anti-HIV-1 treatment using the quantity of an HIV-1 RNA-DNA hybrid in a sample as an indicator, comprising: at least one primer pair consisting of a downstream primer having a sequence complementary to a portion of the nucleotide sequence of the constituent RNA of the HIV-1 RNA-DNA hybrid and an upstream primer having a sequence complementary to a portion of the nucleotide sequence of the constituent DNA of the HIV-1 RNA-DNA hybrid; and restriction enzyme by which double-stranded DNA containing the same nucleotide sequence as DNA extended by the primer pair can be cleaved at any specific site in the nucleotide sequence.

2. The kit according to Clam 1, wherein the kit further comprises a known quantity of DNA capable of competing with DNA extended by the primer pair.

3. A method for DNA amplification by extending DNA using an HIV-1 RNA-DNA hybrid as a template and with at least one primer pair consisting of a downstream primer having a sequence complementary to a portion of the nucleotide sequence of the constituent RNA of the HIV-1 RNA-DNA hybrid and an upstream primer having a sequence complementary to a portion of the nucleotide sequence of the constituent DNA of the HIV-1 RNA-DNA hybrid, comprising the steps of digesting double-stranded DNA present in a sample with a restriction enzyme by which double-stranded DNA containing the same nucleotide sequence as DNA extended by the primer pair can be cleaved at any specific site in the nucleotide sequence and then extending the DNA with the primer pair to achieve DNA amplification.

4. A method for quantification of an HIV-1 RNA-DNA hybrid in a sample, comprising the steps of amplifying DNA by the method recited in Claim 3 and then isolating and detecting the amplified DNA.
